# EUROPÄISCHE PATENTSCHRIFT

(11) **EP 2 111 895 B1**
(45) Veröffentlichungstag und Bekanntmachung des Hinweises auf die Patenterteilung: **25.05.2016**
(21) Anmeldenummer: 09156325.4
(22) Anmeldetag: 26.03.2009
(51) Int. Cl.: A61N 1/375

(54) **Anschlussgehäuse für ein elektromedizinisches Implantat**
Connection housing for an electromedical implant
Boîtier de connexion pour un implant électromédical

(30) Priorität: 26.04.2008 DE 102008021064
(43) Veröffentlichungstag der Anmeldung: 28.10.2009
(73) Patentinhaber: Biotronik CRM Patent AG, 6341 Baar (CH)
(72) Erfinder: Ruschel, Marina, 12679 Berlin (DE); Lehmann, Stefan, 16269 Wriezen (DE)
(74) Vertreter: Galander, Marcus

(56) Entgegenhaltungen:
- US-A- 6 006 135
- US-A1- 2002 138 114
- US-A1- 2003 163 171
- US-A1- 2007 111 587

## Beschreibung

Die Erfindung betrifft ein Anschlussgehäuse, auch Header genannt, für elektromedizinische Implantate, wie beispielsweise Herzstimulatoren, zu denen implantierbare Herzschrittmacher und/oder Cardioverter/Defibrillator zählen, Nerven- oder Gehirnstimulatoren, implantierbare Hörgeräte oder dergleichen.

Derartige elektromedizinische Implantate besitzen üblicherweise ein in der Regel metallisches Hohlgehäuse, welches eine Batterie sowie elektronische Komponenten des elektromedizinischen Implantats einschließt. Mit dem Hohlgehäuse des betriebsbereiten elektromedizinischen Implantats fest verbunden ist ein Anschlussgehäuse - auch Header genannt - mit Steckbuchsen, die als Kontaktbuchsen der Aufnahme und elektrischen Kontaktierung von Elektrodenleitungssteckern dienen. Solche Elektrodenleitungsstecker finden sich am proximalen Ende einer Elektrodenleitung, die in ihrem implantierten Zustand mit ihrem distalen Ende bis an eine zu stimulierende Stelle des Körpers wie in eine oder mehrere Herzkammern des Herzens oder an eine zu stimulierende Nerven- oder Gehirnregion eines Patienten ragt und dort Stimulations- bzw. Defibrillationselektroden aufweist. Die Elektroden sind elektrisch mit entsprechenden Kontakten des Elektrodenleitungssteckers verbunden. Um eine weitere Verbindung mit der Elektronik im Inneren des Hohlgehäuses des elektromedizinischen Implantats herzustellen, weist das Anschlussgehäuse des Implantats in den Kontaktbuchsen entsprechende elektrische Kontaktelemente auf, die der elektrischen Kontaktierung von entsprechenden Gegenkontakten des Elektrodenleitungssteckers dienen. Die elektrischen Kontaktelemente in den Kontaktbuchsen des Anschlussgehäuses sind ihrerseits wieder elektrisch mit der Elektronik im Inneren des Hohlgehäuses des elektromedizinischen Implantats verbunden. Dies geschieht regelmäßig über sogenannte Gehäusedurchführungen, die in der Schnittstelle zwischen Hohlgehäuse und Anschlussgehäuse angeordnet sind.

An das Anschlussgehäuse eines elektromedizinischen Implantats werden hohe Anforderungen gestellt, wobei dieses Anschlussgehäuse in mehrfacher Hinsicht eine hohe Zuverlässigkeit bieten muss. Zum einen muss die elektrische Verbindung zwischen den elektrischen Kontakten in dem Anschlussgehäuse und der Elektronik im Hohlgehäuse des elektromedizinischen Implantats zuverlässig sein. Darüber hinaus muss selbstverständlich auch die elektrische Kontaktierung zwischen den elektrischen Kontakten im Anschlussgehäuse und den entsprechenden Gegenkontakten eines Elektrodenleitungssteckers zuverlässig sein. Weiterhin werden hohe Anforderungen an die Durchschlagfestigkeit und die Dichtigkeit der jeweiligen Kontaktbuchsen gestellt. Darüber hinaus muss der Aufbau des Anschlussgehäuses so sein, dass er eine zuverlässige und sichere Montage genauso erlaubt wie einen daran anschließenden dauerhaft zuverlässigen Betrieb.

Um Elektrodenleitungen verschiedener Hersteller mit elektromedizinischen Implantaten anderer Hersteller verbinden zu können, wurden entsprechende Industriestandards entwickelt, die viele Eigenschaften, insbesondere die Geometrie von Elektrodenleitungssteckern und entsprechend der zugehörigen Kontaktbuchsen im jeweiligen Anschlussgehäuse, festlegen. Ein bisher weit verbreiteter Standard bei Herzstimulatoren ist unter der Bezeichnung IS-1 bekannt. Eine Beschreibung des Standards findet sich in DIN EN 50077. Ein neuerer Standard ist unter der Bezeichnung IS-4 bekannt. Eine Beschreibung dieses Standards ist unter dem Normentwurf ISO TC 150/SC6NXXX zu finden.

Die Existenz von mindestens zwei unterschiedlichen Standards führt zu noch höheren Anforderungen an die Gestaltung eines Anschlussgehäuses für elektromedizinisches Implantat, wenn dieses Implantat gegebenenfalls nicht nur den einen oder den anderen Standard, sondern möglicher Weise auch beide Standards parallel unterstützen soll.

Die hohen Anforderungen an das Anschlussgehäuse beispielsweise eines Herzstimulators haben dazu geführt, dass immer wieder neue Konzepte für ein derartiges Anschlussgehäuse entwickelt werden. Beispiele finden sich in US 7,083,474, US 2005/0137642, US 2007/0100386 und US 2007/0111587.

Weitere Beispiele finden sich in US 2002/138114 A1, US 2003/163171 A1, US 2007/111587 A1 und US 6 006 135 A, wobei US 6 006 135 A ein Anschlussgehäuse nach dem Oberbegriff von Anspruch 1 offenbart. Die Erfindung ist in Anspruch 1 definiert. Bevorzugte Ausführungsbeispiele sind in den abhängigen Ansprüchen definiert.

Die hier beschriebene Erfindung hat zum Ziel, ein Anschlussgehäuse für elektromedizinisches Implantat anzugeben, das den vorgenannten Anforderungen an ein solches Anschlussgehäuse in den vielen Aspekten weitestgehend gerecht wird und insbesondere einen zuverlässigen Aufbau in Verbindung mit verschiedenen Varianten der Anordnung von Kontaktbuchsen gewährleistet.

Diese Aufgabe wird durch ein Anschlussgehäuse für elektromedizinisches Implantat gelöst, das Kontaktbuchsen zur Aufnahme und elektrischen Kontaktierung von Elektrodenleitungssteckern aufweist und ein Grundmodul besitzt, in welches ein separat vorgefertigtes Deckelmodul mit einer Kontaktbuchse nach einem ersten Standard, vorzugsweise entsprechend dem IS-4 Standard, eingesetzt ist, welches im Ergebnis fest und dicht mit dem Grundmodul verbunden ist.

Das Grundmodul kann dabei keine, eine oder mehrere Kontaktbuchsen gemäß einem zweiten Standard aufweisen, welche vorzugsweise dem IS-1 Standard entsprechen.

Das Deckelmodul kann separat vom übrigen Anschlussgehäuse, d. h. insbesondere vom Grundmodul, als Standardteil für verschiedene Varianten des Anschlussgehäuses vorgefertigt werden und auf diese Weise gleichbleibend die besonders hohen Anforderungen erfüllen, die der erste Standard stellt. Dieses betrifft insbesondere die durch entsprechende Abdichtelemente herzustellende Durchschlagfestigkeit zwischen verschiedenen Kontaktelementen.

Das Grundmodul besitzt eine ursprünglich seitlich offene Aufnahmemulde zur Aufnahme des Deckelmoduls, in die das Deckelmodul des fertig montierten Anschlussgehäuses eingesetzt ist. Es hat sich gezeigt, dass eine seitlich offene Aufnahmemulde einer Lösung vorzuziehen ist, bei der ein dem Deckelmodul entsprechendes Modul für eine Kontaktbuchse gemäß dem ersten Standard in Kontaktbuchsen-Längsrichtung in eine entsprechende Aufnahmeöffnung eines Grundkörpers einzusetzen ist. Diese Vorteile betreffen insbesondere die sichere Kontaktierung der Kontaktelemente im Inneren des Deckelmoduls.

In diesem Sinne ist es besonders bevorzugt, wenn das Deckelmodul metallische Verdrahtungsbänder aufweist, die Kontaktelemente im Inneren eines Aufnahmeraums des Deckelmoduls bilden oder mit diesem elektrisch verbunden sind und die fest mit einem Deckelrohling des Deckelmoduls verbunden sind und im vormontierten Zustand des Deckelmoduls freiliegende Enden besitzen.

Vorzugsweise sind die Verdrahtungsbänder durch Umspritzen bei Herstellung des Deckelrohlings fest mit diesem verbunden und bezüglich des Deckelrohlings fixiert.

Der Deckelrohling des Deckelmoduls ist vorzugsweise ein frei fallendes Spritzgussteil aus Kunststoff, welches einen in Längsrichtung des Deckelrohlings verlaufenden Aufnahmeraum aufweist, der im Ergebnis die Kontaktbuchse gemäß des ersten Standards definiert und im fertig montierten Zustand die Kontaktelemente der Kontaktbuchse trägt. Besonders bevorzugt ist es, wenn der Deckelrohling eine seitlich in den Aufnahmeraum mündende Durchtrittsöffnung besitzt, in die eine Steckeraufnahme als Kontaktelement für ein distales Ende eines Elektrodenleitungssteckers gemäß einer dem ersten Standard entsprechenden Norm eingesetzt ist.

In Bezug auf das Grundmodul ist es bevorzugt, wenn dieses eine axiale Durchgangsbohrung aufweist, die einerseits an einer Stirnseite des Grundmoduls mündet und andererseits mit dem Aufnahmeraum des Deckelmoduls fluchtet, wenn das Deckelmodul in die Aufnahmemulde des Grundmoduls betriebsfertig eingesetzt ist.

In den Aufnahmeraum des Deckelmoduls sind vorzugsweise Federhülsen mit Federelementen als elektrische Kontaktelemente und isolierende Distanzhülsen in Längsrichtung einander abwechselnd eingesetzt. Dies kann entweder dadurch geschehen, dass die Federhülsen und Distanzhülsen jeweils einzeln in den Aufnahmeraum eingesetzt werden. Alternativ können die Federhülsen und Distanzhülsen auch auf einer Aufnahme magazinartig angeordnet sein und können dann mit Hilfe der Aufnahme in einem Schritt gleichzeitig in den Aufnahmeraum des Deckelmoduls eingeführt werden.

Der Deckelrohling weist vorzugsweise seitliche Öffnungen auf, über die Kontaktbereiche der metallischen Verdrahtungsbänder frei zugänglich sind und die so angeordnet sind, dass ein jeweiliger Kontaktbereich an eine jeweilige Federhülse unmittelbar angrenzt. Auf diese Weise können die Kontaktbereiche der metallischen Verdrahtungsbänder durch Punktschweißen mit den Federhülsen elektrisch und mechanisch dauerhaft verbunden werden.

Das Anschlussgehäuse ist vorzugsweise Bestandteil eines elektromedizinischen Implantats, insbesondere eines implantierbaren Herzstimulators, wie beispielsweise einem Herzschrittmacher oder Cardioverter/Defibrillator.

Weitere bevorzugte Ausführungsvarianten ergeben sich aus der Kombination der hier erwähnten Merkmale und sind im Übrigen in der nachfolgenden Beschreibung eines Ausführungsbeispiels genannt.

Die Erfindung soll nun anhand eines Herzstimulators mit Kontaktbuchsen im IS-1- und IS-4-Standard mit Bezug auf die Figuren näher erläutert werden. Diese zeigen in
- Figur 1:: einen Herzstimulator mit erfindungsgemäßem Anschlussgehäuse;
- Figur 2:: den Herzstimulator aus Fig. 1 in einer Darstellung, in der Teile des Anschlussgehäuses nach Art einer Explosionszeichnung wiedergegeben sind;
- Figur 3:: ein Deckelmodul für das Anschlussgehäuse in einer Explosionsdarstellung; und
- Figur 4:: eine Darstellung des Deckelmoduls zur Erläuterung der Lage metallischer Anschlussbänder.

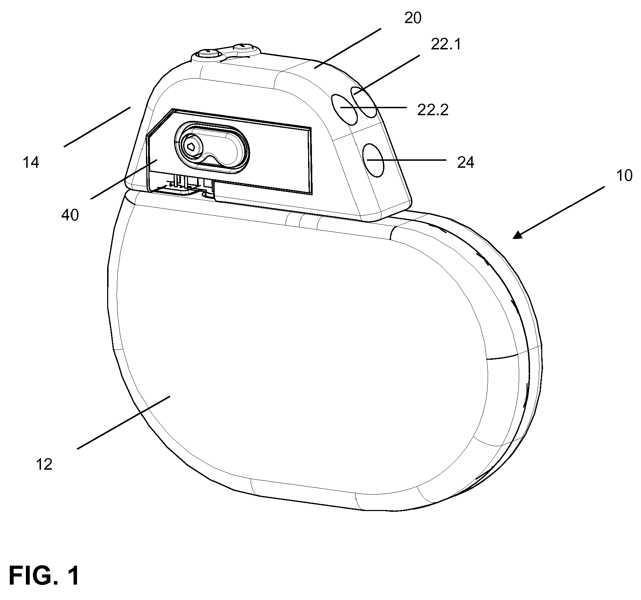

Der in Fig. 1 dargestellte Herzstimulator 10 besitzt ein metallisches Hohlgehäuse 12 und ein mit diesem verbundenes Anschlussgehäuse 14, welches auch als Header bezeichnet wird. In dem Hohlgehäuse 12 befindet sich eine Batterie sowie die elektronischen Komponenten des Herzstimulators 10. Das Anschlussgehäuse 14 besitzt insgesamt drei Kontaktbuchsen 22.1, 22.2 und 24 zur Aufnahme von Elektrodenleitungssteckern von Elektrodenleitungen. Dabei sind die Kontaktbuchsen 22.1 und 22.2 gemäß dem Standard IS-1 gestaltet, während die Kontaktbuchse 24 dem Standard IS-4 entspricht.

Das Anschlussgehäuse 14 weist ein Grundmodul 20 und ein Deckelmodul 40 auf. Die Kontaktbuchsen gemäß IS-1 Standard, 22.1 und 22.2, sind fester Bestandteil des Grundmoduls 20, während die Kontaktbuchse 24 gemäß IS-4 Standard durch das Deckelmodul 40 realisiert ist. Das Deckelmodul 40 beinhaltet dementsprechend alle Bauteile für die Kontaktbuchse 24 gemäß IS-4 Standard. Hingegen sind im Grundkörper 20 ausschließlich Bauteile für Kontaktbuchsen gemäß IS-1 Standard montiert. Abweichend von der Darstellung in Fig. 1 können unterschiedliche Grundmodule 20 vorgesehen sein, die beispielsweise keine Kontaktbuchse gemäß IS-1 Standard aufweisen, sondern nur eine durch das Deckelmodul 40 realisierte Kontaktbuchse gemäß IS-4 Standard, oder auch nur eine Kontaktbuchse gemäß IS-1 Standard und eine Kontaktbuchse gemäß IS-4 Standard. Die unterschiedlichen Varianten des Anschlussgehäuses 14 unterscheiden sich in jenen Fällen ausschließlich durch die Gestaltung des Grundmoduls 20, während das Deckelmodul 40 zur Realisierung der Kontaktbuchse 24 gemäß IS-4 Standard jeweils identisch ist.

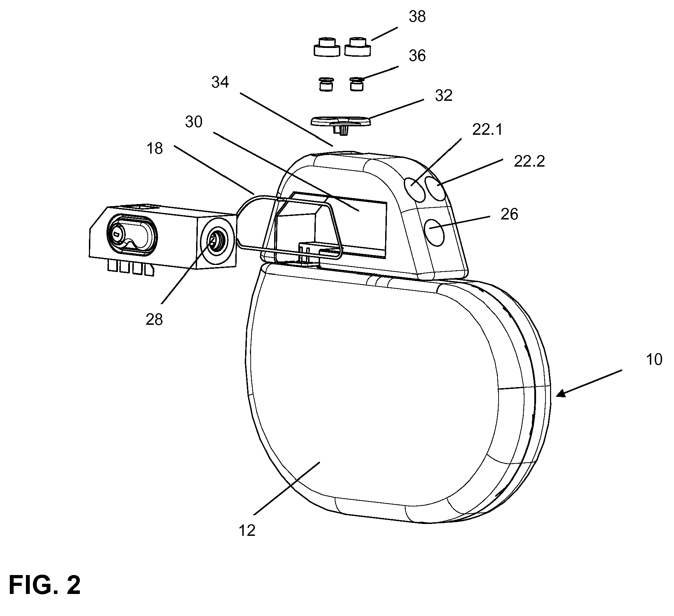

Wie Fig. 2 zu entnehmen ist, besitzt das Grundmodul 20 eine seitlich zunächst offene Aufnahmemulde 30, in die das Deckelmodul 40 eingesetzt ist. Ebenfalls kann bei entsprechenden Ausführungsvarianten eine Antenne 18 in die Aufnahmemulde 30 eingesetzt sein. In dieser Ausführungsvariante ist die Antenne 18 ein Draht, der in einer definierten Fläche ausgebildet ist und diese umspannt. Im eingesetzten Zustand erstreckt sich diese ebene Fläche vorzugsweise parallel zur Kontaktbuchse 24 und umspannt eine möglichst große Fläche im Header. Durch diese Positionierung wird eine gute Sendeleistungen der Antenne ermöglicht.

Wie bereits zuvor erwähnt unterscheidet sich der Aufbau der Baugruppe Grundköper 20 in Abhängigkeit davon, ob der Herzstimulator mit einer, zwei oder drei Elektrodenleitungen verbunden werden soll und entsprechend ein Einkammer-, Zweikammer- oder Dreikammer-Herzschrittmacher ist.

In allen Fällen weist der Grundkörper 20 ein frei fallendes Kunststoffteil auf, das vorzugsweise im Spritzgussverfahren aus Polyurethan hergestellt ist. Für die Variante, in der das Anschlussgehäuse 14 nur eine Kontaktbuchse gemäß IS-4 Standard aufweist, besitzt der Grundkörper 20 keine weiteren Metallteile.

Alternativ zum Spritzgussverfahren kann der Grundkörper 20 auch im Vakuumverguss hergestellt werden. Dies gilt insbesondere für die Variante, dass der Grundkörper 20 selbst keine Kontaktbuchsen gemäß IS-1 Standard aufweist.

Der Grundkörper 20 in dieser einfachsten Ausführungsvariante besitzt neben der seitlich offenen Aufnahmemulde 30 eine axiale Durchgangsbohrung 26, die mit einem Aufnahmeraum 46 des Deckelmoduls 40 fluchtet und es erlaubt, einen Elektrodenleitungsstecker gemäß IS-4 Standard durch die Durchgangsbohrung 26 in den Aufnahmeraum 46 und damit in die Kontaktbuchse gemäß IS-4 Standard des Deckelmoduls 40 einzusetzen. Durch Zusammenfügen von Grundkörper 20 und Deckelmodul 40 ergibt sich eine vollständige Kontaktbuchse 24 gemäß IS-4 Standard, die alle Funktionen erfüllt. Freiliegende Anschluss-Enden von Verdrahtungsbändern 44.1, 44.2, 44.3 und 44.4 des Deckelmoduls 40 können mit entsprechenden Kontaktpins einer nicht weiter bezeichneten Gehäusedurchführung (siehe Fig. 2) verschweißt werden.

Bei einer Variante, in der das Anschlussgehäuse neben einer Kontaktbuchse 24 gemäß IS-4 Standard auch wenigstens eine Kontaktbuchse 22 gemäß IS-1 Standard aufweist, weist der Grundkörper metallische Anschlussteile für die Kontaktbuchse 22 gemäß IS-1 Standard auf, die in einem zweistufigen Prozess mit Polyurethan umspritzt sind, so dass auf diese Weise der Grundkörper 20 entsteht. Ein entsprechender Prozess ist in DE 10 2006 003 224 beschrieben. Alternativ können der Grundkörper 20 auch für diese Variante mit zwei Kontaktbuchsen 22 und 24 im Vakuumverguss hergestellt werden.

Entsprechend weist der Grundkörper 20 für ein Anschlussgehäuse mit zwei Kontaktbuchsen 22.1 und 22.2 gemäß IS-1 Standard und einer Kontaktbuchse 24 gemäß IS-4 Standard metallische Anschlussteile auf, die entsprechend dem in DE 10 2006 003 224 beschriebenen Verfahren zweistufig mit Polyurethan umspritzt sind und so den Grundkörper 20 bilden. Auch hier kann der Grundkörper alternativ im Vakuumverguss hergestellt werden.

Wird der Grundkörper 20, wie in DE 10 2006 003 224 beschrieben, in einem zweistufigen Spritzgussverfahren hergestellt, dann weist dieser eine Kavität 34 auf, über die pro Kontaktbuchse 22 gemäß IS-1 Standard jeweils eine Klemmschraube 36 einzusetzen und einzuschrauben ist, um den entsprechenden Elektrodenleitungsstecker zu fixieren. Zum Verschließen der Kavität 34 sind ein Dichtstopfen 38 sowie ein Deckel 32 aus Polyurethan vorgesehen. Der Deckel 32 wird dabei mittels Kleber dicht mit dem Grundkörper 20 verbunden.

Die verschiedenen Varianten des Anschlussgehäuses 14 unterscheiden sich somit ausschließlich im jeweiligen Grundkörper 20. Jede der Varianten des Grundkörpers 20 ist so gestaltet, dass der jeweilige Grundkörper 20 das universelle Deckelmodul 40 aufnehmen kann.

In Bezug auf die bereits erwähnte Möglichkeit, in die Aufnahmemulde 30 eine Antenne 18 einzulegen, ist darauf hinzuweisen, dass die sichere Positionierung dieser Antenne 18 durch eine Kontur oder einen Halter entweder im Grundkörper 20 oder im Deckelmodul 40 sichergestellt ist. Freie Enden der Antenne 18 werden an entsprechende Pins der Gehäusedurchführung geschweißt (hier nicht näher dargestellt), wobei das Anschweißen vor oder nach dem Verkleben des Deckelmoduls 40 mit dem Grundkörper 20 erfolgen kann. Dieses Verkleben des Deckelmoduls 40 mit dem Grundkörper 20 erfolgt vorzugsweise mit einem Epoxydharz.

Mit dem Fügen von Deckelmodul 40 und Grundkörper 20 ergibt sich ein Anschlussgehäuse 14 mit einer vollständigen Kontaktbuchse 24 gemäß IS-4 Standard. Diese Kontaktbuchse 24 wird durch die Durchgangsbohrung 26 im Grundkörper 20 sowie dem Aufnahmeraum 46 und die darin eingesetzten Kontaktelemente des Deckelmoduls 40 definiert. Nach dem Fügen von Deckelmodul 40 und Grundkörper 20 können die freien Anschluss-Enden der metallischen Verdrahtungsbänder 44.1, 44.2, 44.3 und 44.4 mit entsprechenden Pins der Gehäusedurchführung verschweißt werden. Ein Anschlussgehäuse 14 mit Antenne besitzt somit in einer bevorzugten Ausführungsvariante drei Verdrahtungsebenen, nämliche eine erste für die Verdrahtung der Anschlüsse der Kontaktbuchsen 22 gemäß IS-1 Standard, eine zweite Verdrahtungsebene für die Antenne 18 und eine dritte Verdrahtungsebene für die Verdrahtungsbänder 44 des die Kontaktbuchse 24 gemäß IS-4 Standard bildenden Deckelmoduls 40.

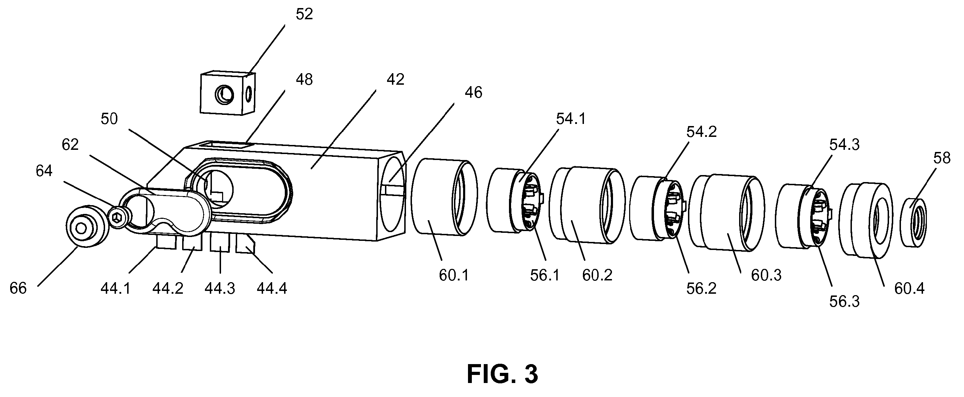

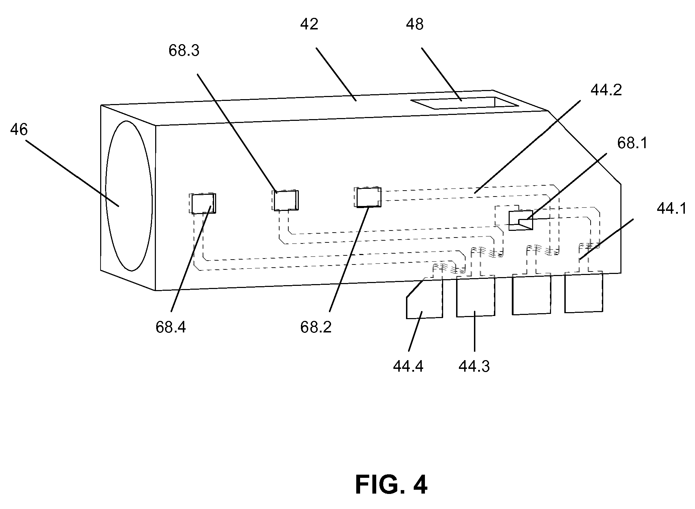

Figuren 3 und 4 zeigen eine Ausführungsform des Deckelmoduls 40 in detaillierterer Darstellung. Ein vollständiges Deckelmodul 40 besitzt eine Anschlussbuchse gemäß IS-4 Standard mit allen metallischen Anschlusselementen und ist vollständig isoliert und abgedichtet. Sämtliche Bestandteile des Deckelmoduls 40 sind in Figur 3 nach Art einer Explosionszeichnung dargestellt. Hauptbestandteil des Deckelmoduls 40 ist ein Deckelrohling 42, der vorzugsweise ein freifallendes Kunststoffteil aus Polyuhrethan ist, das im Spritzgussverfahren hergestellt ist. Dieser Deckelrohling 42 schließt einen in Längsrichtung des Deckelrohlings 42 verlaufenden Aufnahmeraum 46 ein, der eine frontale Öffnung besitzt, in die jeweils dreimal hintereinander eine Distanzhülse 60, und eine Federhülse 54 mit Federkontaktelementen 56 eingesetzt sind, sodass sich auf diese Weise insgesamt drei in Längsrichtung des Aufnahmeraumes hintereinander angeordnete Kontaktelemente der Kontaktbuchse 24 gemäß IS-4 Standart ergeben. Eine vierte Distanzhülse 60.4 und eine Silikondichtung 58 sind als die am weitesten außen gelegenen Bestandteile in den Aufnahmeraum 46 des Deckelmoduls 40 eingesetzt. Die Federhülsen 54 bilden die elektrischen Kontaktelemente der Kontaktbuchse 24 gemäß IS-4 Standard und sind jeweils mit einem Verdrahtungsband 44 elektrisch verbunden. Um diese elektrische Verbindung herzustellen, liegen Kontaktbereiche 68 der metallischen Verdrahtungsbänder in dem Deckelmodul 40 zunächst frei, sodass jeweils eine elektrische Verbindung durch Punktschweißen zwischen den freiliegenden Kontaktbereichen 68 der Verdrahtungsbänder 44 und einer jeweils danebenliegenden Federhülse 54 herzustellen ist. Ein viertes Verdrahtungsband 44.1 dient der Kontaktierung einer Steckeraufnahme 52 die im Bereich des geschlossenen Endes des Aufnahmeraumes 46 des Deckelmoduls 40 angeordnet ist.

Um diese Steckeraufnahme 52 (ein Metallteil) in den Aufnahmeraum 46 des Deckelmoduls 40 einsetzen zu können, besitzt der Deckelrohling 42 eine Durchgangsöffnung 48, die bis in den Aufnahmeraum 46 hineinragt. Durch diese Durchgangsöffnung 48 kann die Steckeraufnahme 52 in den Aufnahmeraum 46 eingesetzt werden und anschließend mit dem Verdrahtungsband 44.1 über dessen frei zugängliches Ende 68.1 durch Punktschweißen verbunden werden.

Alternativ kann die Steckeraufnahme auch vor dem Spritzgießen des Deckelrohlings 42 mit einem Verdrahtungsband wie dem Verdrahtungsband 44.1 durch Schweißen elektrisch verbunden werden und dann zusammen mit diesem Verdrahtungsband und den übrigen Verdrahtungsbändern bei der Herstellung des Deckelrohlings 42 umspritzt werden. Die Durchgangsöffnung 48 ist dann nicht erforderlich.

Auch bei der in den Figuren 3 und 4 dargestellten Ausführungsvariante werden die Verdrahtungsbänder 44.1, 44.2, 44.3 und 44.4 bei der Herstellung des Deckelrohlings 42 mit Polyurethan im Spritzgussverfahren überspritzt. Somit ist die von den Verdrahtungsbändern 44 gebildete Außenverdrahtung in jeder Ausführungsvariante fest mit dem Deckelrohling 42 verbunden und fixiert. Dies führt zu einer erhöhten Zuverlässigkeit.

Nachdem nach Einsetzen der Federhülsen 54 und der Distanzhülsen 60 die Federhülsen 54.1, 54.2 und 54.3 durch Punktschweißen mit den freiliegenden Kontaktbereichen 68.2, 68.3 und 68.4 der Verdrahtungsbänder 44.2, 44.3 und 44.4 verbunden sind, werden die Öffnungen im Deckelrohling 42, über die die freiliegenden Kontaktbereiche 68 der Verdrahtungsbänder 44 zugänglich sind, durch Kleben mittels Polyurethan verschlossen. Gleiches gilt für die Öffnung, die den Zugang zum Kontaktbereich 68.1 des Verdrahtungsbandes 44.1 für die Steckeraufnahme 52 freigibt, sofern diese nicht von vornherein mit in den Deckelrohling 42 eingegossen wird.

Nachdem zunächst der Deckelrohling 42 im Spritzgussverfahren unter Umspritzung der metallischen Anschlussbänder 44 hergestellt worden ist, werden anschließend die Steckeraufnahme 52 durch die Durchgangsöffnung 48 in den Aufnahmeraum 46 eingesetzt. Anschließend oder zuvor werden zunächst eine erste Distanzhülse 60.1, anschließend eine erste Federhülse 54.1, daran anschließend eine zweite Distanzhülse 60.2 und eine zweite Federhülse 54.2 und daran anschließend eine dritte Distanzhülse 60.3 und eine dritte Federhülse 54.3 axial in den Aufnahmeraum 46 eingesetzt. Schließlich wird eine vierte Distanzhülse 60.4 und die Silikondichtung 58 im Bereich des offenen Endes des Aufnahmeraums 46 eingesetzt. Anstelle die verschiedenen Federhülsen 54 und Distanzhülsen 60 einzeln nacheinander axial in den Aufnahmeraum 46 einzusetzen und die Federhülsen 56 dann einzeln mit den freien Anschlussenden 68 der Anschlussbänder 44 zu verschweißen, können die Federhülsen und Dichthülsen gemäß einer alternativen Fertigungsweise magazinartig eventuell auf einer gesonderten Aufnahme angeordnet sein und gemeinsam in einem Schritt in axialer Richtung in den Aufnahmeraum 46 eingesetzt werden. Auf diese Weise entsteht dann ein fertiges Deckelmodul 40, das sämtliche Bestandteile einer Kontaktbuchse 24 gemäß IS-4 Standart aufweist. Deckelmodul 40 und Grundkörper 20 des Anschlussgehäuses 14 können somit völlig unabhängig voneinander hergestellt werden und auch unabhängig voneinander geprüft werden. Erst wenn beide Module diese Prüfung überstanden haben, können sie durch Einsetzen und Verkleben fest miteinander verbunden werden und ergeben das Anschlussgehäuse 14, das dann in der jeweiligen Situation mit dem Hohlgehäuse 12 des implantierbaren Herzstimulators 10 verbunden werden kann.

Das Deckelmodul 40 besitzt im Übrigen eine seitliche Durchgangsöffnung 50, über die eine weitere Klemmschraube 64 einzusetzen ist, die in ein entsprechendes Gewinde in der Steckeraufnahme 52 eingreift und im Betrieb dazu dient, einen in die Kontaktbuchse 24 eingesetzten Elektrodenleitungsstecker sicher zu fixieren. Die seitliche Bohrung 50 kann dann anschließend mittels eines Dichtstopfens 66 und eines Deckels 62 dicht verschlossen werden.

## Patentansprüche

1. Anschlussgehäuse (14) für ein elektromedizinisches Implantat (10) mit Kontaktbuchsen (22.1, 22.2, 24) zur Aufnahme und elektrischen Kontaktierung von Elektrodenleitungssteckern, wobei das Anschlussgehäuse ein Grundmodul (20) aufweist, wobei das Grundmodul (20) wenigstens eine Kontaktbuchse nach einem zweiten Standard aufweist,
und wobei das Anschlussgehäuse ein in das Grundmodul (20) eingesetztes und mit diesem verbundenes, separat vorgefertigtes Deckelmodul (40) mit einer Kontaktbuchse (24) nach einem ersten Standard, aufweist,
**dadurch gekennzeichnet, dass** das Grundmodul (20) eine ursprünglich seitlich offene Aufnahmemulde (30) zur Aufnahme des Deckelmoduls (40) aufweist, in die das Deckelmodul (40) eingesetzt ist und dass das Deckelmodul (40) ein freifallendes Spritzgussteil aufweist, das einen Deckelrohling (42) bildet, der einen in Längsrichtung des Deckelrohlings (42) verlaufenden Aufnahmeraum (46) aufweist, der die Kontaktbuchse gemäß dem IS-4-Standard definiert sowie eine seitlich in dem Aufnahmeraum mündende Durchgangsöffnung (48) aufweist, in die eine Steckeraufnahme (52) eingesetzt ist und dass das Grundmodul (20) eine axiale Durchgangsbohrung (26) aufweist, die in der Aufnahmemulde (30) mündet und mit dem Aufnahmeraum (46) in dem Deckelmodul (40) fluchtet.

2. Anschlussgehäuse nach Anspruch 1, **dadurch gekennzeichnet, dass** das Deckelmodul metallische Verdrahtungsbänder (44.1, 44.2, 44.3, 44.4) aufweist, die Kontaktelemente im Inneren des Aufnahmeraums (46) kontaktieren oder bilden, die fest mit dem Deckelrohling (42) verbunden sind und die im vormontierten Zustand außerhalb des Deckelmoduls frei liegende Ende besitzen.

3. Anschlussgehäuse nach Anspruch 2, **dadurch gekennzeichnet, dass** die metallischen Verdrahtungsbänder (44.1, 44.2, 44.3, 44.4) in den Deckelrohling (42) durch umspritzen integriert und somit fixiert sind.

4. Anschlussgehäuse nach Anspruch 1, **dadurch gekennzeichnet, dass** in den Aufnahmeraum (46) Federhülsen (54.1, 54.2, 54.3) als elektrische Kontaktelemente und isolierende Distanzhülsen (60.1, 60.2, 60.3, 60.4) in Längsrichtung einander abwechselnd eingesetzt sind.

5. Anschlussgehäuse nach Anspruch 3 und 4, **dadurch gekennzeichnet, dass** der Deckelrohling (42) seitliche Öffnungen aufweist, über die Kontaktbereiche (68.1, 68.2, 68.3, 68.4) der metallischen Verdrahtungsbänder (44.1, 44.2, 44.3, 44.4) zunächst frei zugänglich sind und so angeordnet sind, dass ein jeweiliger Kontaktbereich an eine jeweilige Federhülse unmittelbar angrenzt.

6. Anschlussgehäuse nach einem der Ansprüche 1 bis 5, **dadurch gekennzeichnet, dass** der zweite Standard dem IS-1-Standard entspricht.

7. Elektromedizinisches Implantat mit einem Anschlussgehäuse gemäß eines der Ansprüche 1 bis 6.

8. Implantierbarer Herzstimulator mit einem Anschlussgehäuse gemäß eines der Ansprüche 1 bis 6.

## Claims

1. A terminal housing (14) for an electromedical implant (10) having female contacts (22.1, 22.2, 24) to receive and electrically contact electrode line plugs, wherein the terminal housing has a base module (20), wherein the base module (20) has at least one female contact according to a second standard,
and wherein the terminal housing has a separately pre-fabricated cover module (40) that is inserted into the base module (20) and is connected thereto and has a female contact (24) according to a first standard,
**characterised in that** the base module (20) has a receiving trough (30), which is originally open at the side, to receive the cover module (40) and into which the cover module (40) is inserted, and **in that** the cover module (40) has a free-fall injection-moulded part which forms a cover blank (42), which cover blank has a receiving space (46) that runs in a longitudinal direction of the cover blank (42), said receiving space defining the female contact according to the IS-4 standard, and also has a through-opening (48), which leads laterally into the receiving space and into which a plug receptacle (52) is inserted, and **in that** the base module (20) has an axial through-bore (26), which leads into the receiving trough (30) and is aligned with the receiving space (46) in the cover module (40).

2. The terminal housing according to Claim 1, **characterised in that** the cover module has metal wiring bands (44.1, 44.2, 44.3, 44.4), which contact or form contact elements in the interior of the receiving space (46) that are fixedly connected to the cover blank (42) and which have exposed ends in a preinstalled state outside the cover module.

3. The terminal housing according to Claim 2, **characterised in that** the metal wiring bands (44.1, 44.2, 44.3, 44.4) are integrated into the cover blank (42) by overmoulding and are thus fixed.

4. The terminal housing according to Claim 1, **characterised in that** spring sleeves (54.1, 54.2, 54.3) are inserted into the receiving space (46) as electric contact elements and insulating spacer sleeves (60.1, 60.2, 60.3, 60.4) alternating with one another in the longitudinal direction.

5. The terminal housing according to Claim 3 and 4, **characterised in that** the cover blank (42) has lateral openings, via which contact areas (68.1, 68.2, 68.3, 68.4) of the metal wiring bands (44.1, 44.2, 44.3, 44.4) are initially freely accessible and are arranged so that each contact area is directly adjacent to a respective spring sleeve.

6. The terminal housing according to one of Claims 1 to 5, **characterised in that** the second standard corresponds to the IS-1 standard.

7. An electromedical implant having a terminal housing according to one of Claims 1 to 6.

8. An implantable cardiac stimulator having a terminal housing according to one of Claims 1 to 6.

## Revendications

1. Boîtier de connexion (14) pour un implant électromédical (10) avec des contacts (22.1, 22.2, 24) pour la réception et l'établissement d'un contact de fiches de lignes d'électrodes, où le boitier de connexion présente un module de base (20), où le module de base (20) présente au moins un contact selon une deuxième norme,
et où le boitier de connexion présente un module servant de couvercle (40), préfabriqué séparément, inséré dans le module de base (20) et relié avec celui-ci, avec un contact (24) selon une première norme,
**caractérisé en ce que** le module de base (20) présente une cavité de réception (30) à l'origine ouverte latéralement pour la réception du module servant de couvercle (40), dans laquelle le module servant de couvercle (40) est inséré, et que le module servant de couvercle (40) présente une pièce en fonte pour injection tombant librement, qui forme une ébauche de couvercle (42) qui présente un espace de réception (46) s'étendant dans la direction longitudinale de l'ébauche de couvercle (42) qui définit le contact selon la norme IS-4, et présente également une ouverture de passage (48) débouchant latéralement dans l'espace de réception, dans lequel est inséré un support pour fiche (52), et que le module de base (20) présente un alésage pour le passage (26) axial qui débouche dans la cavité de réception (30) et est en alignement avec l'espace de réception (46) dans le module servant de couvercle (40).

2. Boitier de connexion selon la revendication 1, **caractérisé en ce que** le module servant de couvercle présente des bandes de câblage métalliques (44.1, 44.2, 44.3, 44.4), qui sont en contact ou forment les éléments de contact à l'intérieur de l'espace de réception (46), qui sont reliées solidement avec l'ébauche de couvercle (42) et qui possèdent une extrémité libre à l'extérieur du module servant de couvercle à l'état pré-monté.

3. Boitier de connexion selon la revendication 2, **caractérisé en ce que** les bandes de câblage métalliques (44.1, 44.2, 44.3, 44.4) sont intégrées, et ainsi fixées, dans l'ébauche de couvercle (42) par surmoulage.

4. Boîtier de connexion selon la revendication 1, **caractérisé en ce que** des manchons élastiques (54.1, 54.2, 54.3) servant d'éléments de contact électrique et des douilles d'écartement (60.1, 60.2, 60.3, 60.4) sont insérés en alternance dans la direction longitudinale dans l'espace de réception (46).

5. Boitier de connexion selon les revendications 3 et 4, **caractérisé en ce que** l'ébauche de couvercle (42) présente des ouvertures latérales par lesquelles les zones de contact (68.1, 68.2, 68.3, 68.4) des bandes de câblage métalliques (44.1, 44.2, 44.3, 44.4) sont d'abord librement accessibles et sont disposées de telle manière qu'une zone de contact donnée est à la limite immédiate d'un manchon élastique donné.

6. Boitier de connexion selon l'une des revendications 1 à 5, **caractérisé en ce que** la deuxième norme correspond à la norme IS-1.

7. Implant électromédical avec un boitier de connexion selon l'une des revendications 1 à 6.

8. Stimulateur cardiaque implantable avec un boitier de connexion selon l'une des revendications 1 à 6.
